# EUROPEAN PATENT APPLICATION

(11) **EP 1 190 729 A1**
(43) Date of publication of application: **27.03.2002**
(21) Application number: 00308338.3
(22) Date of filing: 22.09.2000
(51) Int. Cl.: A61M 11/00

(54) **Ultrasonic nebulizer**

(71) Applicant: INDUSTRIAL TECHNOLOGY RESEARCH INSTITUTE, Chutung Town, Hsinchu Hsien (TW)
(72) Inventor: Lai, Wen-Pin, Hsinchu, Taiwan 310 (TW); Kio, Ihy-Uan, Hsinchu, Taiwan 310 (TW); Jeng, Jiann-Hwa, Chiung-Lin, Hsinchu,Taiwan 310 (TW)
(74) Representative: Barlow, Roy James

(57) **Abstract**

The invention discloses an improved ultrasonic nebulizer (11) characterized in that a tapered vibration-converging channel (16) is provided in a bottom wall of a nebulizing tank (15) on which a piezoceramic plate (12) is installed such that the channel (16) is capable of focusing the sound pressure. As a result, the sound radiation pressure value in the liquid from the piezoceramic plate (12) of the nebulizer (11) can increase for better nebulizing effect such that the nebulizer (11) consumes less power for nebulizing the same amount of liquid, and assembly of the components is simple and easy.

## Description

### BACKGROUND OF THE INVENTION

### 1. FIELD OF INVENTION

The present invention relates to an improved ultrasonic liquid nebulizer and, in particular, to an ultrasonic nebulizer for nebulizing inhaled therapeutic liquid medicines.

### 2. DESCRIPTION OF THE RELATED ART

The transducers used for the ultrasonic nebulizers are diversified in design depending on different utilization. In general, they are categorized based on resonant frequency, power consumption, nebulizing capacity and the size of the nebulized particles. Two types of transducers are found in currently available nebulizers: a single piezoceramic plate transducer and a mechanical amplitude amplifying transducer. The former is a transducer of the so-called single piezoceramic plate type designed to have a resonant frequency in the range of about several MHz, consume a large amount of power (from tens of watts to about hundreds of watts) and be characterized in large nebulizing capacity and big physical size, but the particles generated are fine, and no filter is required. The latter is a transducer of the so-called mechanical amplitude amplifying transducer designed in different profiles such as Langevin type (Sandwich type), tapered horn type, etc. This type of nebulizer operates at lower resonant frequency (in the range of tens to hundreds KHz), consumes less power (about several watts), and is able to generate nebulized particles which are nearly the same as those generated by the single piezoceramic plate nebulizers in size if a filter is added, but has a much smaller nebulizing capacity. Basically, the former type of nebulizers has the advantages of small particle size and large nebulizing capacity, and the disadvantage of consuming large amount of power. Therefore, it is preferable to improve such nebulizers by lowering power consumption and the overall size so as to make the nebulizers portable.

U.S. Patent 5,217,165 disclosed an improved single piezoceramic plate nebulizer for lowering power consumption and is characterized in that a hollow flat plate is provided on top of the piezoceramic plate, and the diameter of the hollow portion on the flat plate is relevant to the diameter of the electrodes of the piezoceramic plate. The plate is a distance away from the piezoceramic plate such that the power consumed under a certain nebulizing amount can be lowered because of the flat plate, thereby obtaining better nebulizing effect and reducing the power consumption. However, in the above-mentioned patent, the structure and components are considerably complicated and not easy to assemble because of the hollow flat plate.

### SUMMARY OF THE INVENTION

In order to improve the disadvantages of the conventional art, i.e. the components are complicated and uneasy to assemble, an object of the present invention is to provide a small and portable ultrasonic nebulizer wherein the structure is simple and easy to complete.

Another object of the present invention is to provide an ultrasonic nebulizing capable of providing better nebulizing effect and consuming less power.

Still another object of the present invention is to provide an improved ultrasonic nebulizing capable of focusing the sound pressure for liquid nebulization.

To achieve the above objects, the present invention is characterized in that a tapered vibration-converging channel is provided in a bottom wall of a nebulizing tank on which a piezoceramic plate is installed such that the channel is capable of focusing the sound pressure. As a result, the sound radiation pressure value in the liquid from the piezoceramic plate of the nebulizer can increase for better nebulizing effect such that the nebulizer consumes less power for nebulizing the same amount of liquid, and assembly of the components is simple and easy.

The above and other objects, features and advantages will become apparent from the following detailed description of preferred embodiments in conjunction with the accompanying drawings.

### BRIEF DESCRIPTION OF THE DRAWINGS

Fig. 1 is a schematic view showing the structure of an improved ultrasonic nebulizer according to a first preferred embodiment of the present invention.
Fig. 2 is a cross-section view of a tank used in the improved ultrasonic nebulizer shown in Fig. 1.
Fig. 3 is a diagram showing the axial radiation sound pressure distribution in the nebulizing tank for the piezoceramic plate of the improved ultrasonic nebulizer according to the present invention.
Fig. 4 is a schematic view showing the structure of an improved ultrasonic nebulizer according to a second preferred embodiment of the present invention.
Fig. 5 is a schematic view showing the structure of an improved ultrasonic nebulizer according to a third preferred embodiment of the present invention.

### DETAILED DESCRIPTION OF THE INVENTION

The technical concept of the present invention will be better understood from the following description of the preferred embodiments. It should be noted that the embodiments are given by way of illustrating only the preferred structure of the present invention rather than limiting the scope thereof.

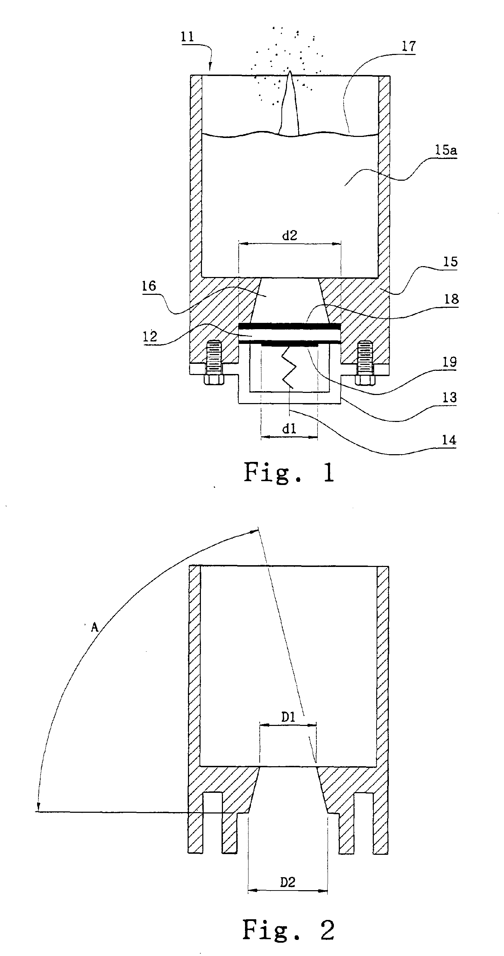

Fig. 1 is a schematic view showing the structure of an improved ultrasonic nebulizer according to a first preferred embodiment of the present invention. As shown in Fig. 1, the ultrasonic nebulizer 11 comprises a piezoceramic plate 12, a piezoceramic plate securing means 13, electrode leads 14, and an nebulizing tank 15 having an cavity 15a upwardly opened and a bottom wall formed with a tapered vibration-converging channel 16.

The piezoceramic plate 12 comprises a PZT material and two circular electrodes 18, 19 on an upper and a lower side surfaces of the PZT material respectively. The upper electrode 18 (the negative electrode) has a diameter d1, and the lower electrode 19 (the positive electrode) has a diameter d2. The piezoceramic plate 12 is secured under the bottom wall of the nebulizing tank 15 by the piezoceramic plate securing means 13 and the upper and the lower electrodes 18, 19 are connected to an outside source (not shown) by the electrode leads 14. In the nebulizing tank 15, the space above the piezoceramic plate 12, including the tapered channel 16 and the cavity 15a, is used for receiving the liquid 17 to be atomized, which can be water, a solution of medicine, an ink, a fuel, etc.

Fig. 2 further depicts the tapered channel 16 in the area close to the piezoceramic plate 12 under the bottom wall of the tank 15. The tapered channel 16 has an upper opening of dimension D1 and a lower opening of dimension D2, and is tapered at an angle of A. Specifically, the dimension D1 of the upper opening is between 0.64dl and 0.78dl, and the lower opening is between 1.21dl and 1.36dl, and the tapering angle is between 63° and 73°.

The operation principle will be further described in the following paragraphs.

After a high frequency (of several MHz) driving power is applied to the ceramic piezoceramic plate 12 through the electrode leads 14 and the electrodes 18 and 19, the ceramic piezoceramic plate 12 will be excited to generate ultrasonic vibration of high frequency and high energy. After being transferred through the liquid 17, the ultrasonic energy will be focused at a certain axial position of the tank 15 due to the function of the tapered channel 16 such that a liquid column above the liquid surface along the axial direction is formed and thus nebulization takes place.

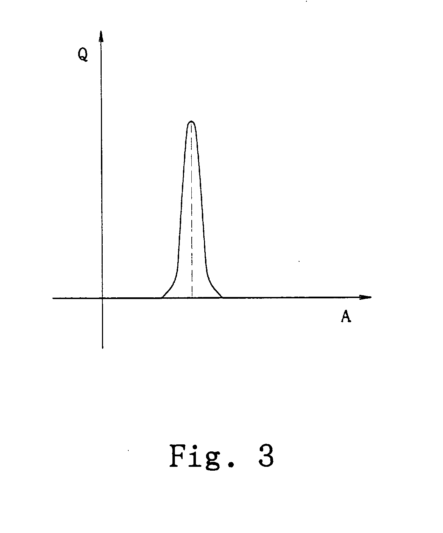

Basically, the above result can be verified experimentally. Fig. 3 is a diagram showing the axial sound radiation pressure distribution in the nebulizing tank for the piezoceramic plate of the improved ultrasonic nebulizer as disclosed in the present invention. The distribution is obtained through measurement made by using a hydrophone which moves along the axial center line of the piezoceramic plate in the nebulizing tank. It can be seen from Fig. 3 that the ultrasonic energy is concentrated in the central axial area of the piezoceramic plate about 1/3 - 1/2 of the axial height of the nebulizing tank, in which area, the liquid is beaten into the shape of a water column, to achieve the above-mentioned effect, and the power consumed is only about 10 watts.

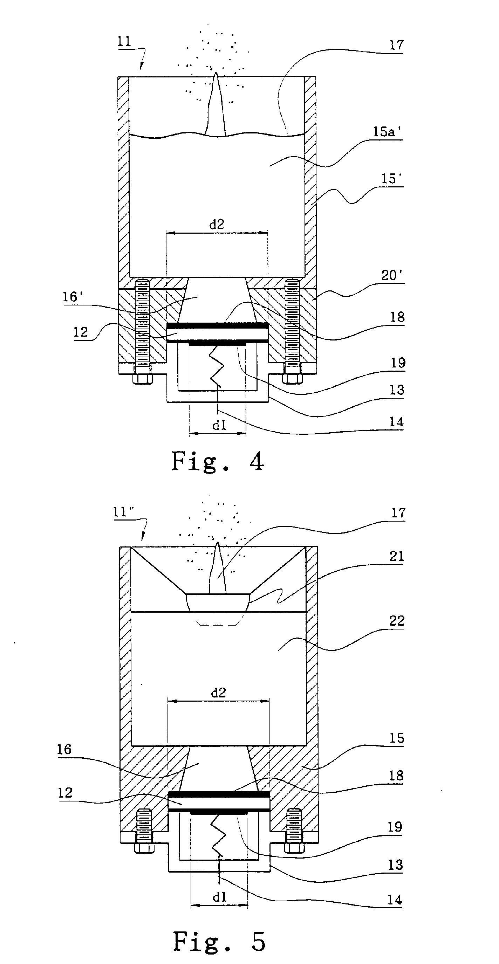

Moreover, in addition to be provided directly in the bottom wall of the tank 15 as disclosed in the first embodiment, the channel 16 can also be provided in a separated vibration-converging block, as disclosed in a second embodiment shown in Fig. 4. In the block 20', a converging channel 16' having an upper small opening and a lower large opening is formed. The tank 15' has a bottom wall formed with a notch 15b' corresponding to the upper opening of the block 20' in size. The same effect can be obtained by having this block 20' attached under the bottom wall of the tank 15' with the notch 15b' aligning with the upper opening of the converging channel 16', and then having the piezoceramic plate 12 secured over the lower opening of the converging channel 16'. The advantage of the second embodiment resides in the changeability in the shape of the converging channel 16' so as to be adapted to different liquids at different height levels.

Fig. 5 shows an ultrasonic nebulizer 11" according to a third embodiment of this invention. In the third embodiment of this invention, the ultrasonic nebulizer 11" comprises a boat 21 in addition to all the components illustrated in the first embodiment. The same components in the first and the third embodiments are indicated with the same reference numerals respectively. However, the nebulizing tank 15 is not used for accumulating the liquid 17 to be nebulized but a media liquid 22 for producing a resonance effect under the function of the tapered channel 16 and the piezoceramic plate 12. The liquid 17 to be nebulized is received in the boat 21.

By means of the tapered channel 16 and the media liquid 22, the media liquid 22 which is located above the tapered channel 16 over the bottom wall of the tank 15 by a specifical height will have a resonance of a largest strength. Such a height is determined by the property of the media liquid 22 and the shape and size of the tapered channel 16. When the boat 21 is located at such a height on the media liquid 22, the largest resonance will be transmitted to the liquid 17 being nebulized via the boat 22. Thus, the liquid 17 is formed with a liquid column over the surface of the liquid 17 and thereby is nebulized. Moreover, since the media liquid 22 is used for transmitting and producing the resonance effect rather than to be nebulized, the media liquid 22 will not lose during nebulization. Thus, the height level of the media liquid 22 would be constant and thus the resonance strength of the nebulizer is maintained at the largest strength. Therefore, an optimal nebulizing effect will be obtained during a whole time of operation in accordance with the third embodiment.

From the foregoing, the tapered channel disclosed in the present invention provides a sound pressure focusing effect such that a liquid column can be created with relatively small power to achieve the nebulizing function. That is, the same nebulizing capacity can be obtained with decreased power. Moreover, the tapered channel is easy to manufacture and assemble, and small in size such that it is possible to implement a compact low-power portable ultrasonic nebulizer having a simple structure which is easy to assemble.

While a preferred embodiment of the present invention is described, it should be understood that various changes and modifications apparent to those skilled in the art are intended to be within the scope as defined in the appended claims.

## Claims

1. An ultrasonic nebulizer, comprising:
a tank having a cavity and a bottom wall formed with a tapered channel which has an upper smaller opening opened to the cavity and a lower larger opening; and
a piezoceramic plate installed on the bottom wall with sealing up the lower larger opening and having an upper electrode and a lower electrode so as to cause liquid accumulated in the tank to resonate under biasing.

2. The ultrasonic nebulizer of Claim 1, wherein the lower electrode has a diameter of dl, the upper smaller opening of the tapered channel has a diameter ranging from 0.64dl to 0.78dl, and the lower larger opening of the tapered channel has a diameter ranging from 1.21dl to 1.36dl.

3. The ultrasonic nebulizer of claim 1, further comprises a boat for receiving liquid to be nebulized, which locates in and contacts with the surface of the liquid accumulated in the tank.

4. An ultrasonic nebulizer, comprising:
a tank having a cavity and a bottom wall formed with a notch of a first diameter,
a vibration-converging block formed with a tapered channel which has an upper smaller opening of a diameter substantially the same to the first diameter and a lower larger opening; and
a piezoceramic plate installed on the vibration converging block with sealing up the lower larger opening and having an upper electrode and a lower electrode so as to cause liquid accumulated in the tank to resonate under biasing.

5. The ultrasonic nebulizer of Claim 4, wherein the lower electrode has a diameter of dl, the upper smaller opening of the tapered channel has a diameter ranging from 0.64dl to 0.78dl, and the lower larger opening of the tapered channel has a diameter ranging from 1.21dl to 1.36dl.

6. The ultrasonic nebulizer of Claim 4, further comprises a boat for receiving liquid to be nebulized, which locates in and contacts with the surface of the liquid accumulated in the tank.
